# EUROPÄISCHE PATENTSCHRIFT

(11) **EP 0 689 822 B1**
(45) Veröffentlichungstag und Bekanntmachung des Hinweises auf die Patenterteilung: **01.10.1997**
(21) Anmeldenummer: 94110255.0
(22) Anmeldetag: 01.07.1994
(51) Int. Cl.: A61H 5/00, A61B 3/024

(54) **Trainingsgerät zur Behandlung von an Wahrnehmungsstörungen leidenden Patienten**
Training device for treating patients suffering from perception disorders
Dispositif d'exercise pour traiter des patients souffrant de troubles de la perception

(43) Veröffentlichungstag der Anmeldung: 03.01.1996
(73) Patentinhaber: Schmielau, Fritz, Prof. Dr.Dr.Dr., D-23556 Lübeck (DE)
(72) Erfinder: Schmielau, Fritz, Prof. Dr.Dr.Dr., D-23556 Lübeck (DE)
(74) Vertreter: Eisenführ, Speiser & Partner

(56) Entgegenhaltungen:
- EP-A- 0 242 723
- WO-A-85/02102
- WO-A-91/07908
- US-A- 4 804 262

## Beschreibung

Die Erfindung betrifft ein Trainingsgerät zur Behandlung von an Wahrnehmungsstörungen leidenden, insbesondere hirngeschädigten Patienten, mit
einer Vielzahl von auf einer Fläche angeordneten Signalgebern zur Erzeugung von Wahrnehmungsreizen,
einem an einer bestimmten, insbesondere zentralen Stelle der Fläche fest angeordneten, während der Behandlung vom Patienten mit seinen Augen zu fixierenden Markierelement,
einer Startsignaleinrichtung zur Erzeugung eines Startsignals,
einer Steuereinrichtung, die einen einzelnen Signalgeber oder eine Gruppe von Signalgebern auswählt und aktiviert,
einer Reaktionserfassungseinrichtung zur Erfassung der Reaktion des Patienten auf die Aktivierung des ausgewählten Signalgebers oder der ausgewählten Signalgebergruppe durch die Steuereinrichtung und
einer Zeiterfassungseinrichtung, die die Reaktionszeit des Patienten zwischen dem Zeitpunkt der Aktivierung des ausgewählten Signalgebers oder der ausgewählten Signalgruppe und dem Zeitpunkt der von der Reaktionserfassungseinrichtung aufgenommenen Reaktion des Patienten mißt.

Eine solche Vorrichtung ist aus der WO-A-85/02102 bekannt.

In der Neuropsychologie besteht ein immer höherer Bedarf nicht nur an einer umfassenden Diagnostik, sondern auch an einer geeigneten Therapie von Wahrnehmungsstörungen. Dies betrifft ganz besonders Patienten, die vor allem nach einem Schlaganfall an Sehstörungen leiden. Dabei ist es wichtig, daß viele Testergebnisse möglichst korrekt interpretiert und therapeutische Maßnahmen auf einem angemessenen Niveau angeboten werden.

Als besonders kritisch für den klinischen Alltag wird der sehr hohe Zeitaufwand der bisher durchgeführten Diagnose und Therapie angesehen. Der Einsatz von bisher bekannten automatisch arbeitenden, rechnergestützten Vorrichtungen zur perimetrischen Gesichtsfeldvermessung wurden wegen häufig erhöhter Ermüdbarkeit der Patienten und möglicher Begleitstörungen als unangemessen insbesondere für den Einsatz bei Gehirnverletzten betrachtet.

Es sind auch kompensatorische Verfahren vorgeschlagen worden, die beispielsweise das Training von sakkadischen Augenbewegungen in das anopische Gesichtsfeld umfassen. Nach anfänglichen Berichten über Erfolge und einer anschließend fehlgeschlagenen Replikation ist diese Methode in den letzten Jahren nicht mehr weiterverfolgt worden.

Es ist daher Aufgabe der Erfindung, ein Trainingsgerät zur Behandlung von an Wahrnehmungsstörungen leidenden, insbesondere hirngeschädigten Patienten zu schaffen, welches eine wirkungsvolle Therapie der Wahrnehmungsstörungen auf einfache Weise ermöglicht.

Gelöst wird diese Aufgabe mit einem Gerät der eingangs genannten Art, welches erfindungsgemäß dadurch gekennzeichnet ist, daß
die Steuereinrichtung zu Beginn eines Betriebszyklus die Startsignaleinrichtung (16) aktiviert, nach Ablauf eines innerhalb bestimmter vorgegebener Grenzen randomisierten ersten Zeitintervalls einen einzelnen Signalgeber oder eine Gruppe von Signalgebern auswählt und aktiviert, nach Ablauf eines zweiten Zeitintervalls nach dem Zeitpunkt der Aktivierung des ausgewählten Signalgebers oder der ausgewählten Signalgebergruppe einen nächsten Betriebszyklus einleitet, wobei die Aktiverungszeitdauer kürzer als das zweite Zeitintervall ist, und in dem nächsten Betriebszyklus oder einem der nächsten Betriebszyklen die Ansteuerung desselben Signalgebers oder derselben Signalgebergruppe wiederholt und/oder einen unmittelbar benachbarten Signalgeber oder eine unmittelbar benachbarte Signalgebergruppe ansteuert, wenn die von der Zeiterfassungseinrichtung gemessene Reaktionszeit kürzer als ein vorgegebenes Minimum oder länger als ein vorgegebenes Maximum ist, welches kürzer als das zweite Zeitintervall ist, oder wenn die von der Reaktionserfassungseinrichtung erfaßte Reaktion des Patienten entsprechend einem zuvor eingegebenen Auswahlkriterium falsch ist.

Das erfindungsgemäße Gerät ermöglicht ein wirkungsvolles und gezieltes Training mit dem Ziel einer funktionellen Restitution von Wahrnehmungsleistungen. Vor allem Patienten mit visuellen Teilfunktionsstörungen oder Schädigungen von auditorischen Funktionen, die als Folge eines Hirninfarkts (Schlaganfall), einer Hirnblutung oder eines Schädelhirntraumas auftreten kann, profitieren von dem erfindungsgemäßen Gerät.

Voraussetzung für die Arbeit mit dem erfindungsgemäßen Trainingsgerät ist die exakte Bestimmung der defekten Bereiche des Wahrnehmungsfeldes. Hierzu wird für die visuelle Modalität üblicherweise ein herkömmliches Perimeter mit im allgemeinen nur im zentralen Gesichtsfeld höherer Präzision verwendet. Für die anschließende Arbeit mit dem vorliegenden erfindungsgemäßen Gerät, mit dem die ermittelten defekten Bereiche des Wahrnehmungsfeldes "wegtrainiert" werden sollen, ist es nicht nur unkritisch, sondern sogar erwünscht, daß der zu behandelnde Patient auf die Lage der ermittelten defekten und zu trainierenden Bereiche innerhalb seines Wahrnehmungsfeldes und/oder die Art der Signalgeberkonfiguration, auf die eine bestimmte Reaktion gewünscht ist, hingewiesen wird. Dadurch wird nämlich der Patient in die Lage versetzt, sich besser auf die defekten Bereiche und Teilfunktionen konzentrieren zu können, was für ein effektives Training eine unabdingbare Voraussetzung ist.
Um das Training im gesamten Wahrnehmungsfeld durchführen zu können, ist eine Vielzahl von Signalgebern auf einer Fläche angeordnet, deren Ausdehnung mindestens so bemessen ist, daß das gesamte Wahrnehmungsfeld des zu behandelnden Patienten abgedeckt wird, wenn der Patient mit seinen Augen eine bestimmte Stelle auf der Fläche fixiert. Diese Stelle enthält ein Markierelement und sollte vorzugsweise etwa in der Mitte der Fläche liegen.

Das erfindungsgemäße Trainingsgerät arbeitet in sukzessiv aufeinanderfolgenden Betriebszyklen, gesteuert von einer Steuereinrichtung, wobei in jedem Betriebszyklus ein bestimmter ausgewählter Signalgeber oder eine bestimmte ausgewählte Signalgebergruppe aktiviert wird. Zu Beginn jedes Betriebszyklus wird von der Steuereinrichtung eine Startsignaleinrichtung aktiviert, die daraufhin ein Startsignal abgibt. Dieses Startsignal, das vorzugsweise einer anderen als der zu trainierenden Sinnesmodalität zugeordnet ist, soll dem Patienten den Hinweis darauf geben, daß ein neuer Betriebszyklus beginnt. Das Startsignal dient somit sozusagen als Warnsignal, mit dem die Aufmerksamkeit des Patienten gesteigert werden soll.

Bevor dann von der Steuereinrichtung ein bestimmter einzelner Signalgeber oder eine bestimmte Gruppe von Signalgebern ausgewählt und aktiviert wird, läßt die Steuereinrichtung zunächst nach Aktivierung der Startsignaleinrichtung ein erstes Zeitintervall verstreichen, das innerhalb bestimmter vorgegebener Grenzen randomisiert ist, d.h. von Betriebszyklus zu Betriebszyklus nach dem Zufallsprinzip unterschiedlich lang innerhalb der gegebenen Grenzen gewählt wird, damit sich der Patient nicht auf feste Einschalt-Zeitpunkte des Signalgebers oder der Signalgebergruppe einstellen kann.

Nach Aktivierung des ausgewählten Signalgebers oder der ausgewählten Signalgebergruppe wird festgestellt, ob und wie der Patient auf den dadurch erzeugten Wahrnehmungsreiz reagiert; hierzu ist eine Reaktionserfassungseinrichtung vorgesehen, die das Auftreten, die Durchführung und die Art der Reaktion erfaßt.

Ferner enthält das erfindungsgemäße Gerät eine Zeiterfassungseinrichtung, die die Reaktionszeit des Patienten zwischen dem Zeitpunkt der Aktivierung des ausgewählten Signalgebers oder der ausgewählten Signalgebergruppe und dem Zeitpunkt der von der Reaktionserfassungseinrichtung aufgenommenen Reaktion des Patienten mißt. Diese Maßnahme ist für das erfindungsgemäße Gerät besonders wichtig, um zu entscheiden, ob der der Stelle des ausgewählten Signalgebers oder der ausgewählten Signalgebergruppe entsprechende Bereich im Wahrnehmungsfeld des Patienten normal funktionsfähig oder in welchem Maße geschädigt ist oder die Wahrnehmungsleistung durch das Training mit dem erfindungsgemäßen Gerät verbessert wurde, so daß ein weiteres Training an dieser Stelle nicht mehr notwendig ist, oder ob wegen nicht erfolgter, zu später oder falscher Reaktion des Patienten das Training in jenem Bereich des Gesichtsfeldes wiederholt und somit noch intensiviert werden muß.

Hierzu ist die Steuereinrichtung erfindungsgemäß so programmiert, daß sie in dem nächsten Betriebszyklus oder einem der nächsten Betriebszyklen die Ansteuerung desselben Signalgebers oder derselben Signalgebergruppe dann wiederholt, wenn die von der Zeiterfassungseinrichtung gemessene Reaktionszeit entweder kürzer als ein vorgegebenes Minimum oder länger als ein vorgegebenes Maximum ist, welches kürzer als das zweite Zeitintervall ist, und/oder wenn die Reaktionserfassungseinrichtung die Art der Reaktion als falsch klassifiziert. Liegt die Reaktionszeit unterhalb des Minimums, so ist davon auszugehen, daß die Reaktion des Patienten auf einem Zufall basiert und somit als fehlerhaft unberücksichtigt zu bleiben hat. Ist dagegen innerhalb der maximal vorgegebenen Zeit keine Reaktion festgestellt worden, so wird dieser Zustand als "ohne Reaktion" gewertet, so daß dann von einem stark geschädigten Bereich im Wahrnehmungsfeld auszugehen ist, welches für die Rehabilitation noch eines stärkeren Trainings bedarf. Im letzteren Fall werden dann derselbe Signalgeber, dieselbe Signalgeber-gruppe, ein unmittelbar benachbarter Signalgeber oder eine unmittelbar benachbarte Signalgebergruppe in einem oder mehreren der nächsten Betriebszyklen wiederholt angesteuert, so daß der defekte Bereich intensiv trainiert wird. Ist nach erfolgreichem Abschluß des Trainings der defekte Bereich des Wahrnehmungsfeldes ausreichend oder völlig rehabilitiert, so wird das Training an einer anderen, noch defekten Stelle des Wahrnehmungsfeldes durch dort wiederholte Ansteuerung des entsprechend zugeordneten Signalgebers oder der entsprechend zugeordneten Signalgebergruppe fortgesetzt. Durch eine solche iterative Vorgehensweise erhält das erfindungsgemäße Gerät adaptive Eigenschaften, indem es das Training im Bereich der defekten Stellen des Wahrnehmungsfeldes automatisch intensiviert, wodurch ein besonders effektives Training mit dem Ziel einer möglichst schnellen funktionellen Restitution von Wahrnehmungsfelddefekten realisierbar.

Dabei ist es besonders vorteilhaft, wenn die Steuereinrichtung während mehrerer Betriebszyklen nacheinander bestimmte dem Grenzbereich zwischen einem gesunden und einem defekten Bereich des Wahrnehmungs- bzw. Wahrnehmungsfeldes des zu behandelnden Patienten zugeordnete Signalgeber oder Signalgeberbaugruppen in einer bestimmten Reihenfolge auswählt und ansteuert, wonach die Signalgeber oder Signalgebergruppen sukzessive in Richtung vom gesunden Bereich in den defekten Bereich angesteuert werden sollten. Wenn die Reaktion des Patienten als falsch klassifiziert wird und/oder die Reaktionszeit nicht innerhalb der zuvor erwähnten Bereiche liegt, können ggf. die Signalgeber oder Signalgebergruppen sukzessive auch wieder in Richtung vom defekten Bereich in den gesunden Bereich angesteuert werden. Mit solchen iterativen Vorgehensweisen ist eine Reduzierung des defekten Bereiches besonders wirkungsvoll erzielbar. Durch das iterative Training wird dabei die Grenze zwischen dem gesunden und dem defekten Bereich allmählich in Richtung des defekten Bereiches verschoben.

Eine besonders einfache Konstruktion der Reaktionserfassungseinrichtung läßt sich dadurch realisieren, daß ein Tastschalter vorgesehen ist, der vom Patienten im Fall einer Wahrnehmung des ausgewählten Signalgebers oder der ausgewählten Signalgebergruppe zu betätigen ist.

Zweckmäßigerweise weist das Markierelement einen Sensor auf, der an die Steuereinrichtung angeschlossen ist und erkennt, ob der Patient das Markierelement mit seinen Augen fixiert oder nicht, wobei im letzteren Fall die Steuereinrichtung den Betrieb des Gerätes so lange unterbricht, bis der Patient seine Augen wieder auf das Markierelement gerichtet hat. Auf diese Weise werden Fehler beim Training durch nicht durchgeführte Fixierung des Markierelementes im wesentlichen unterbunden.

Um den Komfort bei der Behandlung mit dem erfindungsgemäßen Gerät zu erhöhen, was sich letztendlich auch positiv auf die Konzentrationsfähigkeit des Patienten auswirkt, ist eine Stütze zur Halterung des Kopfes des Patienten vorgesehen.

Damit das Training an allen Stellen des Wahrnehmungsfeldes bis in die Peripherie in gleicher Intensität durchgeführt werden kann, sollten die Signalgeber in einer im wesentlichen homogenen Dichte über die Fläche verteilt und vorzugsweise auch in gleichem Abstand voneinander angeordnet sein.

Eine besonders günstige Geometrie ergibt sich, wenn die Fläche mit den darauf angeordneten Signalgebern kugelabschnittsförmig und insbesondere halbkugelförmig ausgebildet ist, wobei der Kopf des zu behandelnden Patienten mit seinen Augen im wesentlichen im Kugelmittelpunkt angeordnet wird.

Der Abstand zwischen den Signalgebern und den Wahrnehmungsorganen des mit seinem Kopf an der Stütze fixierten Patienten sollte vorzugsweise größer als die Armlänge des Patienten betragen. Hierbei kann ggf. auch von einer durchschnittlichen Armlänge ausgegangen werden.

Bei einer gegenwärtig besonders bevorzugten Ausführung bestehen die Signalgeber aus vorzugsweise punktförmigen Lichtquellen zur Erzeugung optischer Signale, die ggf. auch noch unterschiedliche Farben haben und/oder in einer bestimmten Konfiguration, z.B. längs einer variabel orientierten Linie, angeordnet sind.

Alternativ ist es aber auch denkbar, die Signalgeber als Schallwandler zur Erzeugung akustischer Signale auszubilden, um das auditorische Wahrnehmungsvermögen bestimmen und trainieren zu können.

Mit der Steuereinrichtung kann ein Speicher gekoppelt sein, in den ein Algorithmus zur automatischen Ansteuerung von einzelnen Signalgebern oder Signalgebergruppen abspeicherbar ist. Zusätzlich könnte mit der Steuereinrichtung auch eine Eingabeeinrichtung gekoppelt sein, wodurch die Signalgeber wahlweise auch manuell vom Bediener auswählbar sind und somit selektiv angesprochen werden können.

Weitere bevorzugte Ausführungen der Erfindung sind in den Unteransprüchen angegeben.

Nachfolgend wird ein bevorzugtes Ausführungsbeispiel der Erfindung anhand der beiliegenden Zeichnungen beschrieben. Es zeigen:
- Figur 1: eine Draufsicht auf einen Teil des erfindungsgemäßen Gerätes;
- Figur 2: ein schematisches Blockschaltbild des Gerätes;
- Figur 3: ein Zeitdiagramm, das die Arbeitsweise des Gerätes darstellt; und
- Figur 4: einen Ausschnitt aus dem Lichtquellenfeld mit schematischer Darstellung der Betriebsweise einer bevorzugten Ausführung des Gerätes.

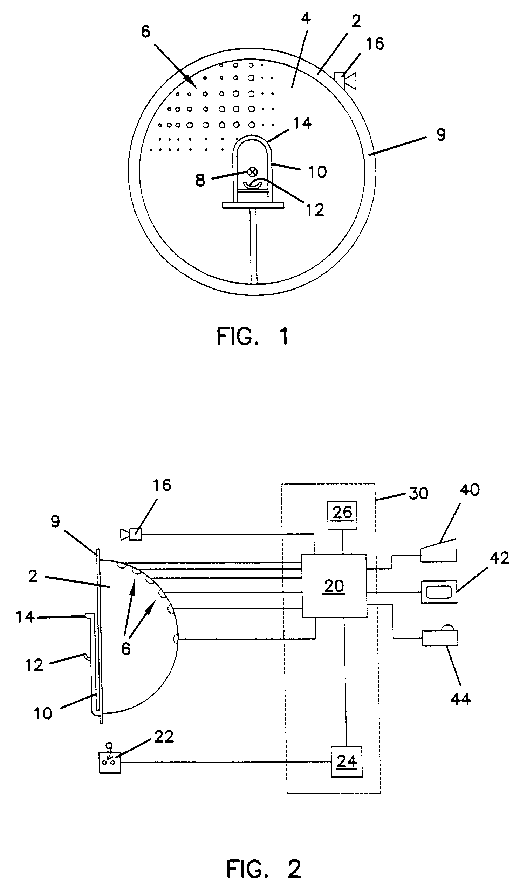

Wie die Figuren 1 und 2 erkennen lassen, weist das dort dargestellte Trainingsgerät zur Erzielung einer funktionellen Restitution von Wahrnehmungsfelddefekten eine Halbkugel 2 auf, an deren Innenfläche 4 eine Vielzahl von Lichtquellen 6 angeordnet sind. In Figur 1, die eine Innenansicht auf die Innenfläche 4 zeigt, sind aus Gründen der Übersichtlichkeit die Lichtquellen 6 nur in einem Teil der Fläche 4 dargestellt; tatsächlich sind sie jedoch in einer im wesentlichen homogenen Dichte über die Fläche 4 verteilt und vorzugsweise auf Hyperbellinien liegend angeordnet. Mit einer möglichst hohen Dichte an Lichtquellen 6 bis an den Rand der Fläche 4 und somit in die Wahrnehmungsfeldperipherie läßt sich ein besonders wirkungsvolles Training mit dem Ziel der funktionellen Restitution von Wahrnehmungsfelddefekten durchführen.

Bevorzugt sind die Lichtquellen 6 als Leuchtdioden ausgebildet. Es ist gleichwohl aber auch möglich, andere Arten von Lichtquellen zu verwenden oder beispielsweise anstelle der Lichtquellen Löcher in der Fläche 4 auszubilden und dort die Enden von Lichtleitern zu befestigen.

Ebenfalls sei an dieser Stelle darauf hingewiesen, daß die Erfindung nicht auf die dargestelle Halbkugel beschränkt ist, sondern die die Lichtquellen tragende Fläche auch eine andere Gestalt haben und beispielsweise plan sein kann.

Ferner ist es mit dem vorliegenden Gerät denkbar, anstelle von visuellen Wahrnehmungsfelddefekten auditorische Wahrnehmungsstörungen zu behandeln. Für diesen Fall müssen anstelle der Lichtquellen 6 akustische Schallwandler verwendet werden. Für diesen Anwendungsfall könnte es beispielsweise zweckmäßig sein, das Training in einem im wesentlichen geschlossenen, schallgedämpften Gehäuse vorzunehmen, an dessen Innenfläche die akustischen Signalgeber angeordnet sind; ein solches Gehäuse könnte beispielsweise auch aus einer Halb- oder Vollkugel bestehen.

In der Mitte der Fläche 4 ist ein Markierelement 8 angebracht, das der Patient während der Behandlung mit seinen Augen zu fixieren hat. In dem Markierelement 8 ist ein Sensor enthalten, der erkennt, ob der Patient das Markierelement 8 mit seinen Augen tatsächlich fixiert oder nicht, und entsprechende Signale abgibt.

Die Halbkugel 2 steht normalerweise auf einem Gestell, und zwar derart, daß die vom umlaufenden Rand 9 gebildete Ebene sich im wesentlichen vertikal erstreckt. Das Gestell ist in den Figuren nicht dargestellt.

An der Halbkugel 2 und/oder dem (nicht dargestellten) Gestell ist eine Kopfstütze 10 befestigt, die eine schalenförmige Kinnstütze 12 und ein bügelförmiges Stirnanlageelement 14 aufweist. Die Kopfstütze 10 ist mit der Kinnstütze 12 und dem Stirnanlageelement 14 so angeordnet, daß der Patient sich mit seinen Augen etwa im Mittelpunkt einer Vollkugel, von der die Halbkugel 2 ein Teil ist, befindet und in das Innere der Halbkugel 2 auf die Fläche 4 mit den Lichtquellen 6 schaut und dabei das Markierelement 8 fixieren kann, wenn der Patient mit seinem Kinn auf der Kinnstütze 12 aufliegt und mit seiner Stirn das Stirnanlageelement 14 berührt. Demnach ist die Kopfstütze 10 etwa in der vom umlaufenden Rand 9 der Halbkugel 2 aufgespannten Ebene oder in unmittelbarer Nähe zu dieser angeordnet. Der Abstand der Lichtquellen 6 von den Augen des mit seinem Kopf an der Kopfstütze 10 fixierten Patienten ist im allgemeinen nicht kleiner als die Armlänge des Patienten.

An der Halbkugel 2 ist außerdem noch ein akustischer Signalgeber 16 angebracht, wie Figur 1 erkennen läßt.

In Figur 2 ist ein Blockschaltbild mit den wesentlichen Komponenten des Trainingsgerätes dargestellt.

Das Gerät weist eine Steuereinrichtung 20 auf, an die die Lichtquellen 6 angeschlossen sind. Aus Gründen der Übersichtlichkeit ist auch in Figur 2 nur ein Teil der Lichtquellen 6 mit ihren zur Steuereinrichtung 20 führenden Leitungen dargestellt. Außerdem sind an die Steuereinrichtung 20 auch noch der Sensor im Markierelement 8 und der akustische Signalgeber 16 angeschlossen.

Ferner ist ein Taster 22 vorgesehen, der in erreichbarer Nähe des Patienten angeordnet ist. Er kann aber auch lose an einem Kabel vorgesehen sein. Während der Behandlung betätigt der Patient den Taster 22 stets dann, wenn er das Licht von einer Lichtquelle 6 oder einer Lichtquellenkonfiguration wahrgenommen hat. Der Taster 22 ist an eine Zeiterfassungseinrichtung 24 angeschlossen, die wiederum über eine Leitung mit der Steuereinrichtung 20 kommuniziert. Alternativ können auch ein Taster mit Wahlmöglichkeit oder mehrere Taster vorgesehen sein, wodurch eine differenzierte Erfassung der Reaktion des Patienten hinsichtlich des Erkennens von verschiedenen orientierten Lichtquellenkonfigurationen möglich ist.

Mit der Steuereinrichtung 20 ist auch ein Speicher 26 gekoppelt, in den beispielsweise ein Algorithmus für den Betrieb der Steuereinrichtung 20 abgespeichert werden kann.

Üblicherweise sind die Steuereinrichtung 20, die Zeiterfassungseinrichtung 24 und der Speicher 26 in einem Mikrocomputer 30 enthalten, der in Figur 2 als Block mit einer unterbrochenen Linie schematisch angedeutet ist.

Zur Eingabe von Daten und zur manuellen Beeinflussung des Betriebes der Steuereinrichtung 20 ist an diese im dargestellten Ausführungsbeispiel eine Tastatur 40 angeschlossen. Zur Ausgabe von Daten und Betriebsergebnissen sind ebenfalls ein Monitor 42 und ein Drucker 44 an die Steuereinrichtung 20 angeschlossen.

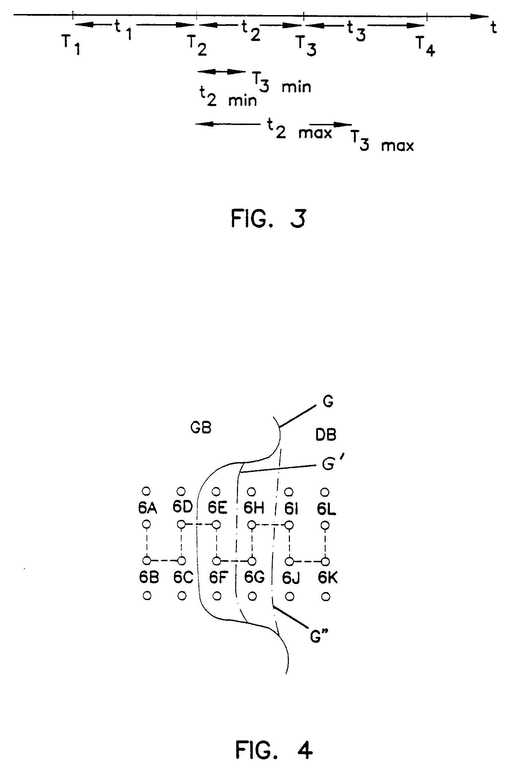

Nachfolgend wird insbesondere anhand der Figuren 3 und 4 die Funktionweise des beschriebenen Gerätes erläutert.

Bevor die Behandlung des Patienten mit dem Trainingsgerät beginnt, müssen die defekten Bereiche des Wahrnehmungsfeldes zunächst genau ermittelt werden, was gewöhnlicherweise mit herkömmlichen Perimetern geschieht. Kennt man dann die Lage und Ausprägung der Wahrnehmungsfelddefekte, beginnt das Training mit dem Gerät. Hierzu muß der Patient seinen Kopf an der Kopfstütze 10 in der zuvor beschriebenen Weise fixieren, wobei sich der Patient zweckmäßigerweise in einer möglichst entspannten Haltung auf einen Stuhl setzt. Außerdem wird der Patient über die Lage, Art und Ausprägung seiner Wahrnehmungsfelddefekte, das zu trainierende Teilgebiet und/oder die visuelle Teilfunktion in Kenntnis gesetzt, damit er sich während der Behandlung auf jene Bereiche und visuelle Teilfunktionen besonders gut konzentrieren kann, wodurch das Training gefördert werden kann.

Das Training wird in aufeinanderfolgenden Betriebszyklen durchgeführt, die von der Steuereinrichtung 20 gesteuert werden. Zu Beginn jedes Betriebszyklus steuert die Steuereinrichtung 20 den akustischen Signalgeber 16 kurz an, damit dieser ein akustisches Warnsignal abgibt, wodurch dem Patienten der Beginn dieses Betriebszyklus angezeigt wird. Gemäß dem in Figur 3 gezeigten Zeitdiagramm beginnt der Betriebszyklus bei T₁. Anschließend verstreicht ein innerhalb bestimmter vorgegebener Grenzen randomisiertes erstes Zeitintervall t₁. D.h. das Zeitintervall t₁ ist von Betriebszyklus zu Betriebszyklus nach einem Zufallsprinzip veränderbar. Erst nach Ablauf des randomisierten ersten Zeitintervalls t₁ werden die Lichtquellen 6 angesteuert. Der Grund für das randomisierte erste Zeitintervall t₁ liegt darin, daß der Patient sich nach Ertönen des akustischen Signalgebers 16 nicht an einen festen Zeitablauf gewöhnen kann, sondern gleichsam von der dann angesteuerten Lichtquelle "überrascht" wird. Nur so sind vernünftige Ergebnisse zu erzielen.

Nach Ablauf des ersten Zeitintervalls t₁ wählt die Steuereinrichtung 20 entweder eine einzelne Lichtquelle oder eine Gruppe von Lichtquellen aus und aktiviert sie zu einem Zeitpunkt T₂. Welche Lichtquelle 6 oder Lichtquellengruppe ausgewählt wird, bestimmt ein im Speicher 26 abgespeicherter Algorithmus. Es ist aber auch möglich, den Algorithmus zu unterbrechen oder deaktivieren und stattdessen über die Tastatur 40 manuell eine bestimmte Lichtquelle 6 oder Lichtquellengruppe selektiv anzusteuern.

Nach Aufleuchten der ausgewählten Lichtquelle oder Lichtquellengruppe ist der Patient aufgefordert, durch Betätigung oder Nicht-Betätigung des Tasters 22 zu signalisieren, ob er den Lichtreiz wahrgenommen hat oder nicht bzw. - bei Wahlmöglichkeit - welche Konfiguration er erkannt hat. Hat er den Lichtreiz wahrgenommen, drückt er den Taster 22. Dies geschieht zum Zeitpunkt T₃ nach Ablauf einer gewissen Verzögerungszeit t₂ nach dem Einschalten der ausgewählten Lichtquelle oder Lichtquellengruppe zum Zeitpunkt T₂. Allerdings muß die Reaktion des Patienten durch Druck auf den Taster 22 innerhalb einer Zeitverzögerung t₂ erfolgen, die länger als ein vorgegebenes Minimum t₂ₘᵢₙ und kleiner als ein vorgegebenes Maximum t₂ₘₐₓ ist. Drückt der Patient den Taster 22 bereits bei t₂ < t₂ₘᵢₙ, so muß es sich hier um einen Zufall handeln, da der Wert von t₂ₘᵢₙ auf die mindest mögliche Reaktionszeit eines Menschen ausgelegt ist. In einem solchen Fall wird das Ergebnis von der Steuereinrichtung 20 als negativ bewertet. Drückt dagegen der Patient den Taster 22 nach Ablauf einer Verzögerungszeit t₂ > t₂ₘₐₓ oder reagiert der Patient überhaupt nicht, so wird das Ergebnis von der Steuereinrichtung 20 ebenfalls als negativ bewertet.

In den beiden vorgenannten "Negativ"-Fällen trifft die Steuereinrichtung 20 die Entscheidung, in einem nächsten Betriebszyklus die Ansteuerung derselben Lichtquelle oder Lichtquellengruppe wie im vorliegenden Betriebszyklus zu wiederholen oder eine andere unmittelbar benachbarte Lichtquelle oder Lichtquellengruppe anzusteuern. Dieser Vorgang wird während nachfolgender Betriebszyklen so lange durchgeführt, bis der Patient einen Lichtreiz der entsprechenden Lichtquelle oder Lichtquellengruppe erkennt. Zwar hat sich während des Versuchsbetriebs des erfindungsgemäßen Gerätes herausgestellt, daß die Anzahl solcher Vorgänge während einer Sitzung nicht beliebig hoch sein kann, da die Konzentrationsfähigkeit der Patienten begrenzt ist; jedoch konnten mit einem solchen iterativen Verfahren über mehrere Sitzungen die Wahrnehmungsfelddefekte reduziert werden.

Hat der Patient bei Wahlmöglichkeit den falschen Taster gedrückt, trifft die Steuereinrichtung 20 die Entscheidung, ob in einem nächsten Betriebszyklus dieselbe oder eine andere Lichtquellenkonfiguration in demselben Gesichtsfeldbereich angesteuert wird. Bei Betätigung des richtigen Tasters wird in einem nächsten Betriebszyklus dieselbe oder eine andere Lichtquellengruppe mit derselben oder einer anderen Lichtquellenkonfiguration angesteuert.

Das Gerät ist hinsichtlich der Auswahl der Lichtquellen 6 durch die Steuereinrichtung 20 frei programmierbar. Es können also verschiedene Algorithmen zur Anwendung kommen, wonach die Steuereinrichtung 20 die Lichtquellen 6 in verschiedenen Reihenfolgen und Mustern nacheinander ansteuert. Das gleiche gilt auch für die Art und Weise der Wiederholung; es ist denkbar, beispielsweise stets dieselbe Lichtquelle oder Lichtquellengruppe iterativ anzusteuern oder iterativ immer eine bestimmte Reihenfolge von Lichtquellen oder Lichtquellengruppen zu durchlaufen, bis der Wahrnehmungsfelddefekt "wegtrainiert" ist.

Ein bevorzugter Algorithmus ist in Figur 4 dargestellt. Hier wählt die Steuereinrichtung 20 während mehrerer Betriebszyklen Lichtquellen 6A bis 6K aus, die in einem Bereich zwischen einem gesunden Bereich GB und einem defekten Bereich DB des Wahrnehmungsfeldes des zu behandelnden Patienten liegen. Dabei steuert die Steuereinrichtung 20 die dargestellten ausgewählten Lichtquellen 6A bis 6L in einer "mäanderförmigen" Folge nacheinander an, und zwar derart, daß die Lichtquellen sukzessive in Richtung vom gesunden Bereich GB in den defekten Bereich DB angesteuert werden und somit die Lichtreize vom gesunden Bereich GB in den defekten Bereich "laufen". Zuerst werden also die Lichtquellen 6A bis 6D im gesunden Bereich GB nacheinander angesteuert und anschließend die Lichtquellen 6E bis 6L im defekten Bereich DB. Diese über 12 Betriebszyklen dauernde Sequenz wird so lange wiederholt, bis sich die Grenze G zwischen dem gesunden Bereich GB und dem defekten Bereich DB in Richtung auf den defekten Bereich DB verschiebt und der defekte Bereich DB somit reduziert wird. An diesem Beispiel, auf das die Erfindung nicht notwendigerweise beschränkt ist, läßt sich also deutlich das Prinzip des erfindungsgemäßen Trainingsgerätes erkennen, wonach durch wiederholtes "Vortasten" in den defekten Bereich DB dieser letztendlich "wegtrainiert" und somit reduziert wird.

Ein effektives Training wird jedoch nur dann erzielt, wenn der Patient während der Sitzung stets das Markierelement 8 (vgl. Figuren 1 und 2) fixiert. Sollte dies einmal nicht der Fall sein, so übermittelt der im Markierelement 8 enthaltene Sensor ein entsprechendes Signal an die Steuereinrichtung 20, die daraufhin den Betrieb so lange unterbricht, bis der Patient seine Augen wieder auf das Markierelement 8 gerichtet hat. Ggf. ertönt dabei auch ein Warnsignal über den akustischen Signalgeber 16.

Wie Figur 3 außerdem erkennen läßt, schließt sich an den Zeitpunkt T₃ noch eine zweite Verzögerungszeit t₃ an, bevor zum Zeitpunkt T₄ der nächstfolgende Betriebszyklus eingeleitet wird. Die zweite Verzögerungszeit t₃ kann ggf. auch variabel und randomisierbar eingestellt werden.

Die Trainingsergebnisse können auf dem Monitor 42 angezeigt und über den Drucker 44 ausgedruckt werden. Ferner ist es möglich, die Trainingsergebnisse statistisch auszuwerten.

Abschließend sei noch darauf hingewiesen, daß es für einige Anwendungen vorteilhaft sein könnte, verschiedenfarbige Lichtreize zu erzeugen und auch die Helligkeit des Lichtes zu steuern.

## Patentansprüche

1. Trainingsgerät zur Behandlung von an Wahrnehmungsstörungen leidenden, insbesondere hirngeschädigten Patienten, mit
einer Vielzahl von auf einer Fläche (4) angeordneten Signalgebern (6) zur Erzeugung von Wahrnehmungsreizen,
einem an einer bestimmten, insbesondere zentralen Stelle der Fläche (4) fest angeordneten, während der Behandlung vom Patienten mit seinen Augen zu fixierenden Markierelement (8),
einer Startsignaleinrichtung (16) zur Erzeugung eines Startsignals,
einer Steuereinrichtung (20), die einen einzelnen Signalgeber (6) oder eine Gruppe von Signalgebern (6) auswählt und aktiviert,
einer Reaktionserfassungseinrichtung (22) zur Erfassung der Reaktion des Patienten auf die Aktivierung des ausgewählten Signalgebers (6) oder der ausgewählten Signalgebergruppe durch die Steuereinrichtung (20) und
einer Zeiterfassungseinrichtung (24), die die Reaktionszeit (t₂) des Patienten zwischen dem Zeitpunkt (T₂) der Aktivierung des ausgewählten Signalgebers (6) oder der ausgewählten Signalgruppe und dem Zeitpunkt (T₃) der von der Reaktionserfassungseinrichtung (22) aufgenommenen Reaktion des Patienten mißt,
dadurch gekennzeichnet, daß
die Steuereinrichtung (20) zu Beginn (T₁) eines Betriebszyklus die Startsignaleinrichtung (16) aktiviert, nach Ablauf eines innerhalb bestimmter vorgegebener Grenzen randomisierten ersten Zeitintervalls (t₁) einen einzelnen Signalgeber (6) oder eine Gruppe von Signalgebern (6) auswählt und aktiviert, nach Ablauf eines zweiten Zeitintervalls (t₂ + t₃) nach dem Zeitpunkt (T₂) der Aktivierung des ausgewählten Signalgebers (6) oder der ausgewählten Signalgebergruppe (6) einen nächsten Betriebszyklus einleitet, wobei die Aktivierungszeitdauer kürzer als das zweite Zeitintervall ist, und in dem nächsten Betriebszyklus oder einem der nächsten Betriebszyklen die Ansteuerung desselben Signalgebers (6) oder derselben Signalgebergruppe wiederholt und/oder einen unmittelbar benachbarten Signalgeber (6) oder eine unmittelbar benachbarte Signalgebergruppe ansteuert, wenn die von der Zeiterfassungseinrichtung (24) gemessene Reaktionszeit (t₂) kürzer als ein vorgegebenes Minimum (t₂ₘᵢₙ) oder länger als ein vorgegebenes Maximum (t₂ₘₐₓ) ist, welches kürzer als das zweite Zeitintervall ist, oder wenn die von der Reaktionserfassungseinrichtung (22) erfaßte Reaktion des Patienten entsprechend einem zuvor eingegebenen Auswahlkriterium falsch ist.

2. Gerät nach Anspruch 1,
dadurch gekennzeichnet, daß die Steuereinrichtung (20) während mehrerer Betriebszyklen nacheinander bestimmte dem Grenzbereich zwischen einem gesunden und einem defekten Bereich (GB, DB) des Wahrnehmungsfeldes des zu behandelnden Patienten zugeordnete Signalgeber (6A bis 6L) oder Signalgebergruppen auswählt und ansteuert.

3. Gerät nach Anspruch 2,
dadurch gekennzeichnet, daß die Steuereinrichtung (20) die Signalgeber (6A bis 6L) oder Signalgebergruppen in einer bestimmten Reihenfolge auswählt und ansteuert, wonach die Signalgeber (6A bis 6L) oder Signalgebergruppen sukzessive in Richtung vom gesunden Bereich (GB) in den defekten Bereich (DB) angesteuert werden.

4. Gerät nach mindestens einem der Ansprüche 1 bis 3,
dadurch gekennzeichnet, daß die Reaktionserfassungseinrichtung einen oder mehrere vom Patienten im Fall einer Wahrnehmung des ausgewählten Signalgebers (6) oder der ausgewählten Signalgebergruppe zu betätigende Tastschalter (22) aufweist.

5. Gerät nach mindestens einem der Ansprüche 1 bis 4,
dadurch gekennzeichnet, daß das zweite Zeitintervall (t₃) variierbar und ggf. randomisierbar ist.

6. Gerät nach mindestens einem der Ansprüche 1 bis 5,
dadurch gekennzeichnet, daß das Markierelement (8) einen Sensor aufweist, der an die Steuereinrichtung (20) angeschlossen ist und erkennt, ob der Patient das Markierelement (8) mit seinen Augen fixiert oder nicht, wobei im letzteren Fall die Steuereinrichtung (20) den Betrieb des Gerätes so lange unterbricht, bis der Patient seine Augen wieder auf das Markierelement (8) gerichtet hat.

7. Gerät nach mindestens einem der Ansprüche 1 bis 6,
gekennzeichnet durch eine Stütze (10) zur Halterung des Kopfes.

8. Gerät nach mindestens einem der Ansprüche 1 bis 7,
dadurch gekennzeichnet, daß die Signalgeber (6) in einer im wesentlichen homogenen Dichte über die Fläche (4) verteilt angeordnet sind.

9. Gerät nach mindestens einem der Ansprüche 1 bis 8,
dadurch gekennzeichnet, daß die Signalgeber (6) etwa in gleichem Abstand voneinander angeordnet sind.

10. Gerät nach mindestens einem der Ansprüche 1 bis 9,
dadurch gekennzeichnet, daß die Fläche (4) kugelabschnittsförmig ausgebildet ist.

11. Gerät nach Anspruch 10,
dadurch gekennzeichnet, daß die Fläche (4) im wesentlichen halbkugelförmig ausgebildet ist.

12. Gerät nach Anspruch 7 sowie Anspruch 10 oder 11,
dadurch gekennzeichnet, daß die Stütze (10) derart angeordnet ist, daß sich der darauf abgestützte Kopf des zu behandelnden Patienten mit seinen Augen im wesentlichen im Kugelmittelpunkt befindet.

13. Gerät nach den Ansprüchen 9 bis 12,
dadurch gekennzeichnet, daß die Signalgeber (6) auf Hyperbellinien liegen.

14. Gerät nach mindestens einem der Ansprüche 1 bis 13, dadurch gekennzeichnet, daß die Signalgeber (6) aus vorzugsweise punktförmigen Lichtquellen zur Erzeugung optischer Signale bestehen.

15. Gerät nach Anspruch 14,
dadurch gekennzeichnet, daß die Lichtquellen verschiedene Farben haben.

16. Gerät nach mindestens einem der Ansprüche 1 bis 13,
dadurch gekennzeichnet, daß die Signalgeber aus Schallwandlern zur Erzeugung akustischer Signale bestehen.

17. Gerät nach mindestens einem der Ansprüche 1 bis 16,
gekennzeichnet durch einen mit der Steuereinrichtung (20) gekoppelten Speicher (26), in den ein Algorithmus zur automatischen Ansteuerung von einzelnen Signalgebern (6) oder Signalgebergruppen abspeicherbar ist.

18. Gerät nach mindestens einem der Ansprüche 1 bis 17,
gekennzeichnet durch eine mit der Steuereinrichtung (20) gekoppelte Eingabeeinrichtung (40), wodurch die Signalgeber (6) wahlweise auch manuell vom Bediener auswählbar sind.

19. Gerät nach mindestens einem der Ansprüche 1 bis 18,
dadurch gekennzeichnet, daß die Steuereinrichtung (20) eine statistische Auswertung der von der Zeiterfassungseinrichtung (24) gelieferten Meßergebnisse vornimmt.

20. Gerät nach mindestens einem der Ansprüche 1 bis 19,
gekennzeichnet durch eine mit der Steuereinrichtung (20) gekoppelte Einrichtung (42, 44) zur Ausgabe von Daten.

21. Gerät nach mindestens einem der Ansprüche 1 bis 20,
dadurch gekennzeichnet, daß mittels der Steuereinrichtung (20) die Intensität der von den angesteuerten Signalgebern (6) erzeugten Wahrnehmungsreize einstellbar ist.

22. Gerät nach mindestens einem der Ansprüche 1 bis 21,
dadurch gekennzeichnet, daß die Startsignaleinrichtung (16) ein akustisches Signal abgibt.

23. Gerät nach mindestens einem der Ansprüche 1 bis 22, dadurch gekennzeichnet, daß der Abstand der Signalgeber (6) von den Wahrnehmungsorganen des Patienten während des Trainings mit dem Gerät nicht geringer als die Armlänge des Patienten ist.

## Claims

1. Training device for the therapy of, in particular, brain-damaged patients having perception defects, comprising
a large number if signalers (6) arranged on one surface (4) for the production of perceptual stimuli,
a marking element (8) that is firmly attached in a specific - and, in particular, central - location on the surface (4) and on which the patient must focus his eyes during the therapy,
a start-signaling device (16) for producing a starting signal,
a control mechanism (20) that selects and activates a single signaler (6) or a signaler group (6) randomized within specific predetermined limits,
a reaction-detecting device (22) for detecting the reaction of the patient to the activation of the selected signaler (6) or the selected signaler group by the control mechanism (20), and
a time-determining device (24) that measures the reaction time (t₂) of the patient between the time (T₂) of the activation of the selected signaler (6) or the selected signal group and the time (T₃) of the patient's reaction recorded by the reaction-detecting device (22),
characterized in that
said control mechanism (20) activates a start-signaling device (16) at the beginning (T₁) of an operating cycle, selects and activates a single signaler (6) or a signaler group (6) after the expiration of a first time interval (t₁) randomized within specific predetermined limits, initiates a subsequent operating cycle, after expiration of a second interval (t₂ + t₃) following the time T₂ of the activation of the selected signaler (6) or the selected signaler group (6), wherein the duration of the activation is shorter than the second time interval, and repeats the activation of the same signaler (6) or signaler group and/or an immediately adjacent signaler (6) or an immediately adjacent signaler group in the next operating cycle, or in one of the subsequent operating cycles, if the reaction time (t₂) measured by the time-determining device (24) is shorter than a predetermined minimum (t₂ₘᵢₙ) or longer than a predetermined maximum (t₂ₘₐₓ) which is shorter than the second time interval or if the reaction of the patient detected by the reaction-detecting device (22) is wrong according to a previously input selection criterion.

2. Device according to claim 1, characterized in that, during several consecutive operating cycles, the control mechanism (20) selects and activates specific signalers (6A to 6L) or signaler groups associated with the border area between a healthy and a defective area (GB, DB) of the perceptual field of the patient in therapy.

3. Device according to claim 2, characterized in that the control mechanism (20) selects and activates the signalers (6A to 6L) or signaler groups in a specific sequence, after which the signalers (6A to 6L) or signaler groups are successively activated in the direction from the healthy area (GB) into the defective area (DB).

4. Device according to at least one of the claims 1-3, characterized in that the reaction-detecting device has one or several push-button switches (22) that have to be pressed by the patient if he perceives the selected signaler (6) or the selected signaler group.

5. Device according to at least one of the claims 1-4, characterized in that the second time interval (t₃) is variable and, optionally, randomizable.

6. Device according to at least one of the claims 1-5, characterized in that the marking element (8) has a sensor that is connected to the control mechanism (20) and recognizes whether the patient fixes his eyes on the marking element (8) or not, whereby, in the latter case, the control mechanism (20) interrupts the operation of the device until the patient has again fixed his eyes on the marking element (8).

7. Device according to at least one of the claims 1-6, characterized by a head support (10).

8. Device according to at least one of the claims 1-7, characterized in that the signalers (6) are distributed over the surface (4) in a basically homogeneous density.

9. Device according to at least one of the claims 1-8, characterized in that the signalers (6) are arranged at approximately the same distance from each other.

10. Device according to at least one of the claims 1-9, characterized in that the surface (4) is developed in the shape of a spherical section.

11. Device according to claim 10, characterized in that the surface (4) is basically developed in the shape of a hemisphere.

12. Device according to claim 7 and to claim 10 or 11, characterized in that the support (10) is arranged in such a manner that the head of the patient, while being supported on it, is basically positioned with the eyes at the center of the sphere.

13. Device according to claims 9-12, characterized in that the signalers (6) are located on hyperbola lines.

14. Device according to at least one of the claims 1-13, characterized in that the signalers consist of, preferably, dot-shaped light sources for producing optical signals.

15. Device according to claim 14, characterized in that the light sources have different colors.

16. Device according to at least one of the claims 1-13, characterized in that the signalers consist of sound transducers for the production of audible signals.

17. Device according to at least one of the claims 1-16, characterized by a memory (26), which is coupled with the control mechanism (20), and in which an algorithm can be stored for the automatic activation of individual signalers (6) or signaler groups.

18. Device according to at least one of the claims 1-17, characterized by an input device (40) that is coupled to the control mechanism (20) and by which the signalers (6) can optionally also be manually selected by the operator.

19. Device according to at least one of the claims 1-18, characterized in that the control mechanism (20) performs a statistical evaluation of the measuring results supplied by the time-determining device (24).

20. Device according to at least one of the claims 1-19, characterized by a device (42, 44), for the output of data, that is coupled to the control mechanism (20).

21. Device according to at least one of the claims 1-20, characterized in that the intensity of the perceptual stimuli produced by the activated signaler (6) can be adjusted by means of the control mechanism (20).

22. Device according to at least one of the claims 1-21, characterized in that the start-signaling device (16) emits an audible signal.

23. Device according to at least one of the claims 1-22, characterized in that the distance of the signalers (6) from the organs of perception of the patient, during the training with the device, is not smaller than the length of the patient's arm.

## Revendications

1. Dispositif d'exercice pour traiter des patients souffrant de troubles de la perception, en particulier de patients ayant une lésion du cerveau, comportant
plusieurs générateurs de signaux (6) disposés sur une surface (4) et destines à générer des stimuli de perception,
un élément marqueur (8) disposé de manière fixe à un point déterminé, notamment à un point central de la surface (4) et devant être fixé des yeux par le patient pendant le traitement,
un dispositif de signal de départ (16) pour générer un signal de départ,
un dispositif de commande (20) qui sélectionne et active un générateur de signaux (6) individuel ou un groupe de générateurs de signaux (6),
un dispositif d'enregistrement des réactions (22) pour enregistrer la réaction du patient lors de l'activation du générateur de signaux (6) sélectionné ou du groupe de générateurs de signaux sélectionné, par le dispositif de commande (20) et
un dispositif d'enregistrement du temps (24) qui mesure le temps de réaction (t₂) du patient entre l'instant (T₂) de l'activation du générateur de signaux (6) sélectionné ou du groupe de signaux sélectionné et l'instant (T₃) de la réaction du patient enregistrée par le dispositif d'enregistrement des réactions (22), caractérisé en ce que
le dispositif de commande (20) au début (T₁) d'un cycle de fonctionnement, active le dispositif de signal de départ (16), en ce qu'après expiration d'un premier intervalle de temps (t₁) randomisée à l'intérieur des limites prédéfinies, il sélectionne et active un générateur de signaux (6) individuel ou un groupe de générateurs de signaux (6) et en ce qu'après expiration d'un second intervalle de temps (t₂ + t₁) après le moment (T₂) de l'activation du générateur de signaux (6) sélectionné ou du groupe de générateurs de signaux (6) sélectionné, il lance un prochain cycle de fonctionnement, la durée de l'activation étant plus courte que le second intervalle de temps et dans le prochain cycle de fonctionnement ou l'un des prochains cycles de fonctionnement reproduisant la commande du même générateur de signaux (6) ou du même groupe de générateurs de signaux et/ou commandant un générateur de signaux (6) directement adjacent ou un groupe de générateur de signaux directement adjacent si le temps de réaction (t₂) mesuré par le dispositif d'enregistrement du temps (24) est plus court qu'un minimum (t₂ₘᵢₙ) prédéfini ou plus long qu'un maximum (t₂ₘₐₓ) prédéfini qui est plus court que le second intervalle de temps ou si la réaction du patient enregistrée par le dispositif d'enregistrement des réactions (22) est fausse conformément à un critère de sélection entré précédemment.

2. Dispositif selon la revendication 1, caractérisé en ce que pendant plusieurs cycles de fonctionnement successifs, le dispositif de commande (20) sélectionne et commande certains générateurs de signaux (6A à 6L) ou groupes de générateurs de signaux affectés à la zone limite entre une zone saine et une zone endommagée (GB, DB) du champ de perception du patient à traiter.

3. Dispositif selon la revendication 2, caractérisé en ce que le dispositif de commande (20) sélectionne et commande les générateurs de signaux (6A à 6L) ou les groupes de générateurs de signaux dans un ordre déterminé selon lequel les générateurs de signaux (6A à 6L) ou les groupes de générateurs de signaux sont commandés successivement dans le sens de la zone saine (GB) à la zone endommagée (DB).

4. Dispositif selon au moins l'une quelconque des revendications 1 à 3, caractérisé en ce que le dispositif d'enregistrement des réactions présente un ou plusieurs commutateurs à touche (22) devant être actionnés par le patient en cas de perception du générateur de signaux (6) sélectionné ou du groupe de générateurs de signaux sélectionné.

5. Dispositif selon au moins l'une quelconque des revendications 1 à 4, caractérisé en ce que le second intervalle de temps (t₃) peut varier et le cas échéant, peut être randomisé.

6. Dispositif selon au moins l'une quelconque des revendications 1 à 5, caractérisé en ce que l'élément marqueur (8) présente un capteur qui est relié au dispositif de commande (20) et détecte si le patient fixe des yeux l'élément marqueur (8) ou pas, dans ce dernier cas, le dispositif de commande (20) interrompant le fonctionnement du dispositif jusqu'à ce que le patient ait à nouveau dirigé les yeux sur l'élément marqueur (8).

7. Dispositif selon au moins l'une quelconque des revendications 1 à 6, caractérisé par un support (10) pour soutenir la tête.

8. Dispositif selon au moins l'une quelconque des revendications 1 à 7, caractérisé en ce que les générateurs de signaux (6) sont disposés selon une densité essentiellement homogène et répartis sur la surface (4).

9. Dispositif selon au moins l'une quelconque des revendications 1 à 8, caractérisé en ce que les générateurs de signaux (6) sont disposés à peu près à égale distance l'un de l'autre.

10. Dispositif selon au moins l'une quelconque des revendications 1 à 9, caractérisé en ce que la surface (4) est conçue en forme de segment sphérique.

11. Dispositif selon la revendication 10, caractérisé en ce que la surface (4) est conçue en substance en forme d'hémisphère.

12. Dispositif selon la revendication 7 et selon la revendication 10 ou la revendication 11, caractérisé en ce que le support (10) est disposé de telle sorte que la tête (niveau des yeux) du patient à traiter qui s'y appuie, se trouve essentiellement au centre de la sphère.

13. Dispositif selon l'une quelconque des revendications 9 à 12, caractérisé en ce que les générateurs de signaux (6) se trouvent sur des lignes hyperboliques.

14. Dispositif selon au moins l'une quelconque des revendications 1 à 13, caractérisé en ce que les générateurs de signaux (6) se composent de sources lumineuses de préférence ponctuelles permettant de générer des signaux optiques.

15. Dispositif selon la revendication 14, caractérisé en ce que les sources lumineuses ont des couleurs différentes.

16. Dispositif selon au moins l'une quelconque des revendications 1 à 13, caractérisé en ce que les générateurs de signaux se composent de transducteurs acoustiques permettant de générer des signaux acoustiques.

17. Dispositif selon au moins l'une quelconque des revendications 1 à 16, caractérisé par une mémoire (26) couplée au dispositif de commande (20) et dans laquelle un algorithme permettant la commande automatique des générateurs de signaux (6) individuels ou des groupes de générateurs de signaux peut être stocké.

18. Dispositif selon au moins l'une quelconque des revendications 1 à 17, caractérisé par un dispositif d'entrée (40) couplé au dispositif de commande (20) et grâce auquel les générateurs de signaux (6) peuvent être aussi sélectionnés au choix manuellement par l'opérateur.

19. Dispositif selon au moins l'une quelconque des revendications 1 à 18, caractérisé en ce que le dispositif de commande (20) effectue une analyse statistique des résultats de mesure fournis par le dispositif d'enregistrement du temps (24).

20. Dispositif selon au moins l'une quelconque des revendications 1 à 19, caractérisé par un dispositif (42, 44) couplé au dispositif de commande (20) et permettant de sortir des données.

21. Dispositif selon au moins l'une quelconque des revendications 1 à 20, caractérisé en ce que l'intensité des stimuli de perception générés par les générateurs de signaux (6) commandés peut être réglé à l'aide du dispositif de commande (20).

22. Dispositif selon au moins l'une quelconque des revendications 1 à 21, caractérisé en ce que le dispositif de signal de départ (16) délivre un signal acoustique.

23. Dispositif selon au moins l'une quelconque des revendications 1 à 22, caractérisé en ce que pendant l'exercice avec le dispositif, la distance entre les générateurs de signaux (6) et les organes de perception du patient n'est pas inférieure à la longueur du bras du patient.
